# EUROPEAN PATENT APPLICATION

(11) **EP 2 535 767 A1**
(43) Date of publication of application: **19.12.2012**
(21) Application number: 11739734.9
(22) Date of filing: 01.02.2011
(51) Int. Cl.: G02F 1/15, C07D 213/22, C08G 79/00, C09K 9/02, E06B 9/24

(54) **SMART WINDOW EMPLOYING ORGANIC/METALLIC HYBRID POLYMER, METHOD FOR MANUFACTURING SMART WINDOW, AND SMART WINDOW SYSTEM**

(30) Priority: 08.02.2010 JP 2010025058
(71) Applicant: National Institute for Materials Science, Tsukuba-shi, Ibaraki 305-0047 (JP)
(72) Inventor: HIGUCHI, Masayoshi, Tsukuba-shi Ibaraki 305-0047 (JP); ZHANG, Jian, Tsukuba-shi Ibaraki 305-0047 (JP)
(74) Representative: Jansen, Cornelis Marinus
(86) International application number: PCT/JP2011/052007
(87) International publication number: WO 2011/096386

(57) **Abstract**

There is provided a smart window having a configuration in which an organic-metallic hybrid polymer and an electrolyte are sandwiched between two sheets of ITO glass;
the configuration facilitates an increase in area and, in addition, after the smart window is made transparent by applying drive voltage thereto, it takes time for the smart window to revert to a colored state due to the memory effect thereof even when application of the drive voltage is stopped, and therefore the configuration has excellent power saving properties; and
therefore, a smart window system without a power source can be configured by means of combination with a solar cell.

## Description

### [Technical Field]

The present invention relates to a so-called smart window (also known as smart glass or the like). More specifically, the present invention relates to a glass capable of actively changing the light transmitting properties through a control signal or the like, unlike a normal light control glass reacting to the intensity of light that is irradiated onto the glass.
Priority is claimed on Japanese Patent Application No. 2010-025058, filed February 8, 2010, the content of which is incorporated herein by reference

### [Background Art]

The technological development of smart windows that is aimed at a variety of applications is underway. For example, application as window glass can be mentioned for purposes, such as to achieve automated curtains or blinds or to save energy consumption for cooling by automatically interrupting the incidence of sunlight. In addition, use as a blind in order to obscure the appearance of the room as needed even inside the building, or use as a projector screen by making a usually transparent partition opaque only when necessary can be mentioned. Further, some products for achieving such functions have already been commercially available.

However, it was difficult to manufacture conventional smart windows having a large area, and they were also expensive. In addition, it is necessary to continuously supply power to the conventional smart windows at all times in order to maintain the desired level of light transmittance, and thus there has been a problem in terms of power consumption.

Among the conventional smart windows, those using a liquid crystal as a light transmittance-variable material are available, and it is possible to reduce the power consumption in this type of smart window. However, in this case, because the interval between transparent electrodes is maintained with a high degree of accuracy when producing a smart window having a large area, high processing accuracy is required, or selection of materials or an increase in the complexity of structures for withstanding the stress during installation and the aging is needed. As a result, the production cost will be very high.

When installing a smart window as window glass in a building, unlike conventional glass, the power needs to be supplied, which is a problem. That is, a large-scale installation becomes necessary because the power lines need to be routed from a commercial power supply to the location where the glass is used. This problem becomes more serious when a smart window is newly introduced in already existing buildings that are designed with no consideration regarding such requirements.

### [Citation List]

### [Patent Documents]

Patent Document 1: PCT International Publication WO 2007/049371
Patent Document 2: Japanese Unexamined Patent Application, First Publication No. 2007-112769
Patent Document 3: Japanese Unexamined Patent Application, First Publication No. 2007-112957
Patent Document 4: PCT International Publication WO 2008/081762
Patent Document 5: Japanese Unexamined Patent Application, First Publication No. 2008-162967
Patent Document 6: Japanese Unexamined Patent Application, First Publication No. 2008-162976
Patent Document 7: Japanese Unexamined Patent Application, First Publication No. 2008-162979
Patent Document 8: PCT International Publication WO 2008/143324
Patent Document 9: Japanese Unexamined Patent Application, First Publication No. 2009-223159
Patent Document 10: Japanese Unexamined Patent Application, First Publication No. 2009-265437

### [Summary of Invention]

### [Technical Problem]

The present invention has an object of providing a smart window having a simple structure with low power consumption which solves the aforementioned problems associated with the prior art. Furthermore, the present invention also has another object of providing a smart window capable of using a solar cell as a power source instead of a commercial power supply by taking advantage of a feature of low power consumption.

### [Solution to Problem]

By adopting a configuration in which an organic-metallic hybrid polymer (OMHP) is sandwiched between transparent electrodes, there is provided a smart window that achieves the above-mentioned object.

According to one aspect of the present invention, a smart window in which the organic-metallic hybrid polymer and an electrolyte are sandwiched between two conductive transparent plates is provided.

For the aforementioned organic-metallic hybrid polymer, polymers represented by the following general formula (I) or (II) can be used. 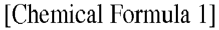 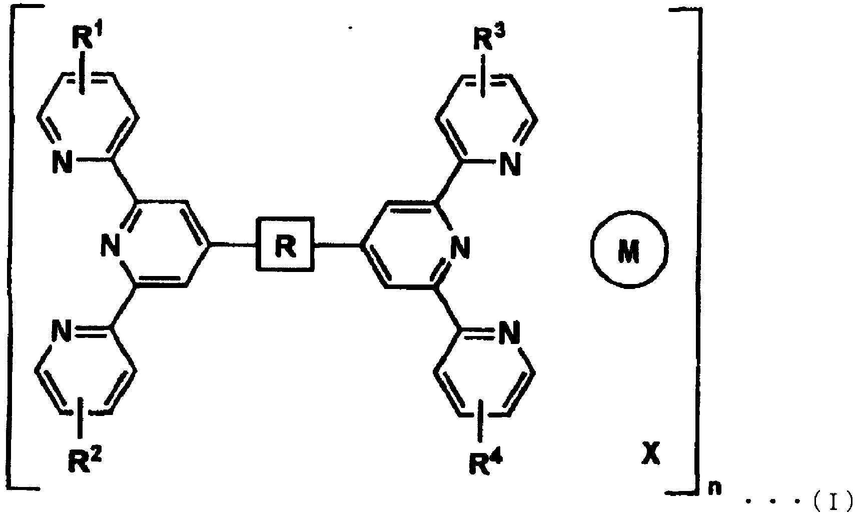 In the formula, M represents a metal ion; X represents a counter anion; R represents a spacer containing a carbon atom and a hydrogen atom, or a spacer directly connecting two terpyridyl groups; each of R¹ to R⁴ independently represents a hydrogen atom or a substituent; and n represents an integer of 2 or more which indicates a degree of polymerization.

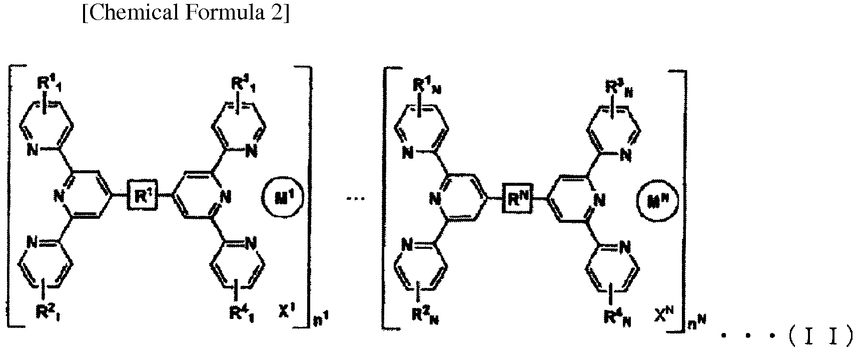

In the formula, each of M¹ to M^{N} (wherein N represents an integer of 2 or more) independently represents a metal ion; each of X¹ to X^{N} (wherein N represents an integer of 2 or more) independently represents a counter anion, each of R¹ to R^{N} (wherein N represents an integer of 2 or more) independently represents a spacer containing a carbon atom and a hydrogen atom, or a spacer directly connecting two terpyridyl groups; each of R¹₁ to R¹_{N}, R²₁ to R²_{N}, R³₁ to R³_{N}, and R⁴₁ to R⁴_{N} (wherein N represents an integer of 2 or more) independently represents a hydrogen atom or a substituent; and each of n¹ to n^{N} (wherein N represents an integer of 2 or more) independently represents an integer of 2 or more which indicates a degree of polymerization.
The above conductive transparent plate may be a glass plate having a conductive thin film formed on the surface thereof. In addition, the above transparent plate may be an ITO glass. Further, the organic-metallic hybrid polymer sandwiched between the aforementioned two transparent plates can be applied on top of the aforementioned transparent plate by spin coating a solution of organic-metallic hybrid polymer on one of the aforementioned two transparent plates. Additionally, the above solution of organic-metallic hybrid polymer may be a solution prepared by dissolving the organic-metallic hybrid polymer in a mixture of methanol and isopropanol. In addition, the aforementioned electrolyte may be a conductive gel. Further, the thickness of the aforementioned electrolyte between the aforementioned two transparent plates may be from 1 mm to 10 mm. Furthermore, the aforementioned electrolyte may contain lithium perchlorate.

According to another aspect of the present invention, a method of producing a smart window is provided, in which the aforementioned electrolyte is applied to both the aforementioned organic-metallic hybrid polymer on top of the aforementioned transparent plate and the ITO film of one of the aforementioned two transparent plates to which the organic-metallic hybrid polymer has not been applied, and the surfaces of the aforementioned two transparent plates to which the aforementioned electrolyte has been applied are combined to adhere, thereby producing the aforementioned smart window.

According to yet another aspect of the present invention, there is provided a smart window system provided with the aforementioned smart window and a drive circuit for intermittently applying a drive voltage to the aforementioned two transparent electrode plates. In addition, a solar cell which supplies power to the aforementioned drive circuit can be provided.

According to yet another aspect of the present invention, there is provided a smart window system including the aforementioned smart window, and a solar cell and a direct current power supply which are connected in parallel and in opposite directions from each other, and in which an external light-sensitive drive unit that supplies a drive signal to the aforementioned smart window is provided, and the transmittance of the aforementioned smart window is changed in the direction so as to counteract the change in the illuminance of light outside.

### [Advantageous Effects of Invention]

The present invention is capable of achieving the above-mentioned objects, as well as providing a smart window having a large area with low power consumption, and configuring a smart window system in combination with a solar cell which does not require power from a commercial power supply at all.

### [Brief Description of the Drawings]

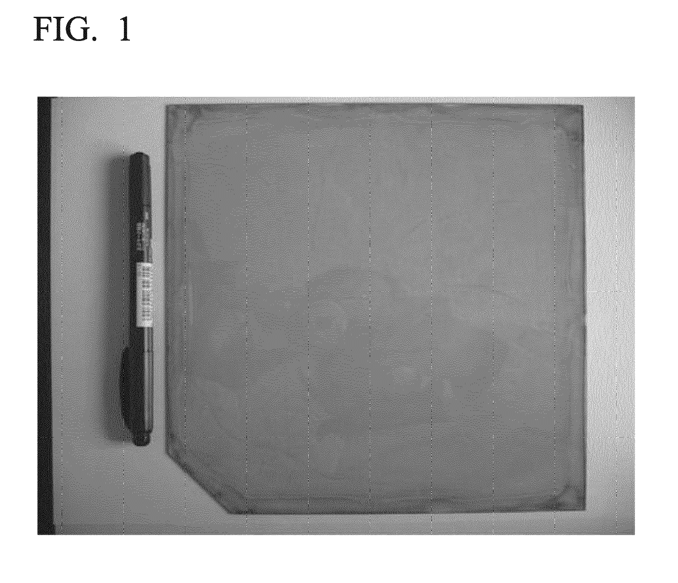

FIG. 1 is a photograph showing an ITO glass prepared in an example of the present invention onto which an organic-metallic hybrid polymer film has been applied.
FIG. 2 is a diagram showing the structure of a smart window in an example of the present invention.
FIG. 3 is a photograph showing the state of the smart window at the time of coloration in an example of the present invention.
FIG. 4 is a photograph showing the state of the smart window at the time of decoloration in an example of the present invention.
FIG. 5 is a graph showing the optical absorption spectra of the smart window in an example of the present invention.
FIG. 6 is a graph showing the relationship between the light transmittance of the smart window and the concentration of the organic-metallic hybrid polymer solution at the time of production in an example of the present invention.
FIG. 7 is a graph showing the changes in the drive voltage and light transmittance of the smart window in accordance with the wavelength in an example of the present invention.
FIG. 8 is a graph showing the change in the light transmittance over time when the smart window is driven in an example of the present invention.
FIG. 9 is a graph showing the change in the light transmittance over time when the smart window is driven in an example of the present invention.
FIG. 10 is a graph showing the memory effect of the smart window in an example of the present invention.
FIG. 11 is a diagram showing in more detail the memory effect of the smart window in an example of the present invention.
FIG. 12 is a diagram showing a long memory effect in another Example of the present invention.
FIG. 13 is a conceptual diagram of a smart window system in an example when the smart window of the present invention is applied to a window of a building or the like.
FIG. 14 is a conceptual diagram of another Example at the time of light irradiation, when a solar cell is used in the smart window system of the present invention.
FIG. 15 is a conceptual diagram of another Example at the time during which light is not irradiated, when a solar cell is used in the smart window system of the present invention.

### [Description of Embodiments]

An organic-metallic hybrid polymer including a metal ion and bisterpyridine has a specific characteristic of color change based on the metal-to-ligand charge transfer (MLCT) absorption. In the present invention, this property of organic-metallic hybrid polymer is applied to a smart window.
In the present description, the term "smart window" refers to a window that can electrically switch the transmission thereof.
Further, the term "organic-metallic hybrid polymer" refers to a polymer prepared by forming a complex between an organic molecule with two terpyridyl groups and a metal ion to thereby have a structure in which organic molecules and metal ions are bonded alternately along the main chain.

The organic-metallic hybrid polymer is a series of polymers represented by the following general formula (I) or (II).

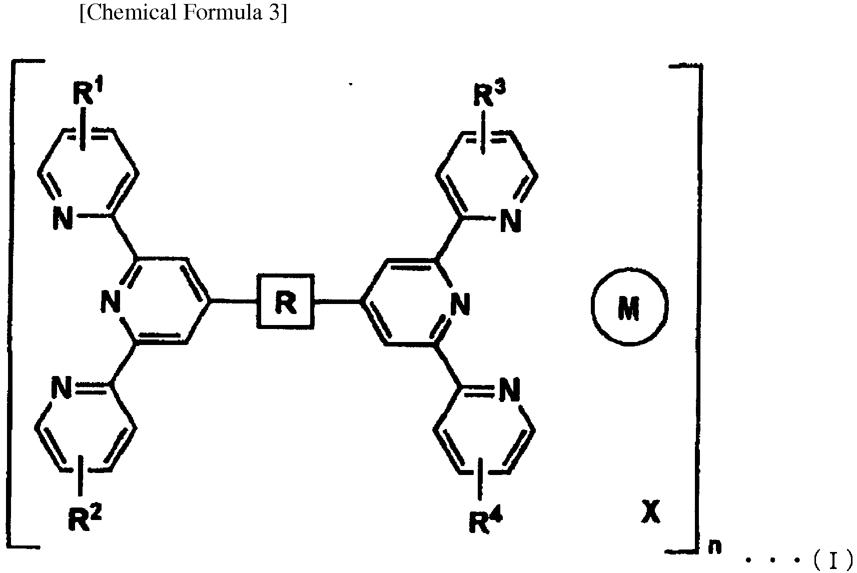

In the formula, M represents a metal ion; X represents a counter anion; R represents a spacer containing a carbon atom and a hydrogen atom, or a spacer directly connecting two terpyridyl groups; each of R¹ to R⁴ independently represents a hydrogen atom or a substituent; and n represents an integer of 2 or more which indicates a degree of polymerization.

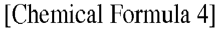

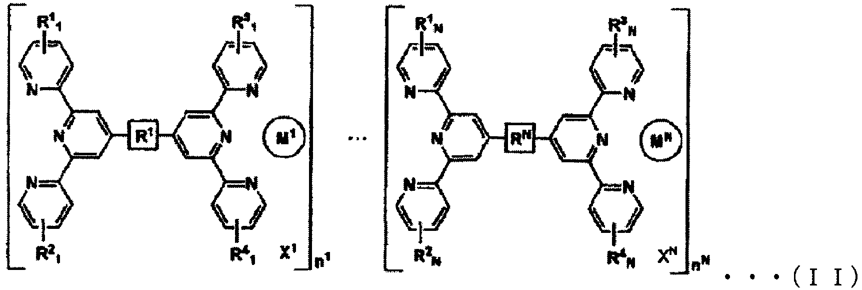

In the formula, each of M¹ to M^{N} (wherein N represents an integer of 2 or more) independently represents a metal ion; each of X¹ to X^{N} (wherein N represents an integer of 2 or more) independently represents a counter anion, each of R¹ to R^{N} (wherein N represents an integer of 2 or more) independently represents a spacer containing a carbon atom and a hydrogen atom, or a spacer directly connecting two terpyridyl groups; each of R¹₁ to R¹_{N}, R²₁ to R²_{N}, R³₁ to R³_{N}, and R⁴₁ to R⁴_{N} (wherein N represents an integer of 2 or more) independently represents a hydrogen atom or a substituent; and each of n¹ to n^{N} (wherein N represents an integer of 2 or more) independently represents an integer of 2 or more which indicates a degree of polymerization.

Further, in any of the above general formula, the metal ion of the organic-metallic hybrid polymer is at least one metal ion selected from the group consisting of an iron ion, a cobalt ion, a nickel ion, a zinc ion, and a ruthenium ion. Furthermore, the counter anion of the organic-metallic hybrid polymer is at least one anion selected from the group consisting of an acetate ion, a chloride ion, a hexafluorophosphate ion, a tetrafluoroborate ion, and a polyoxometalate.

The organic-metallic hybrid polymer represented by the general formula (I) and general formula (II) used in the present invention is constituted of a bis(terpyridine) derivative, a metal ion and a counter anion.

Further, by forming a complex between a bis(terpyridine) derivative exhibiting coordination properties and a metal ion, a polymer complex which is in a state where the bis(terpyridine) derivative and the metal ion are alternately connected is formed.

The organic-metallic hybrid polymer exhibits a color based on the charge-transfer absorption from the metal to the bis(terpyridine) derivative as a ligand. In other words, when the organic-metallic hybrid polymer is electrochemically oxidized, the color of the polymer disappears. On the other hand, when the organic-metallic hybrid polymer in this colorless state is electrochemically reduced, the state of the polymer reverts to the colored state. These phenomena can be caused repeatedly.

R in the general formula (I) and R¹ to R^{N} in the general formula (II) are individually a spacer for connecting two terpyridyl groups. By appropriately selecting the type of spacer, the angle of the pyridyl group in the organic-metallic hybrid polymer can be arbitrarily set, thus enabling material design for the organic-metallic hybrid polymer.

With respect to the spacer, one having two terpyridyl groups directly connected (namely, a single bond) may be used, although a divalent organic group containing a carbon atom and a hydrogen atom can be used. Examples of such divalent organic groups include aliphatic hydrocarbon groups, alicyclic hydrocarbon groups, aromatic hydrocarbon groups and heterocyclic groups. Of these, arylene groups such as a phenylene group and a biphenylene group are preferred. In addition, these divalent organic groups may have a substituent, including an alkyl group such as a methyl group, an ethyl group, or a hexyl group; an alkoxy group such as a methoxy group or a butoxy group; or a halogen atom such as chlorine or bromine. Moreover, such spacers may further contain an oxygen atom or a sulfur atom. Since the oxygen atom or sulfur atom has a modifying ability, it is advantageous for the material design of the organic-metallic hybrid polymer.

Preferred examples of the spacers include divalent arylene groups represented by the following formulae (1) to (11).

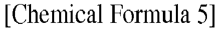

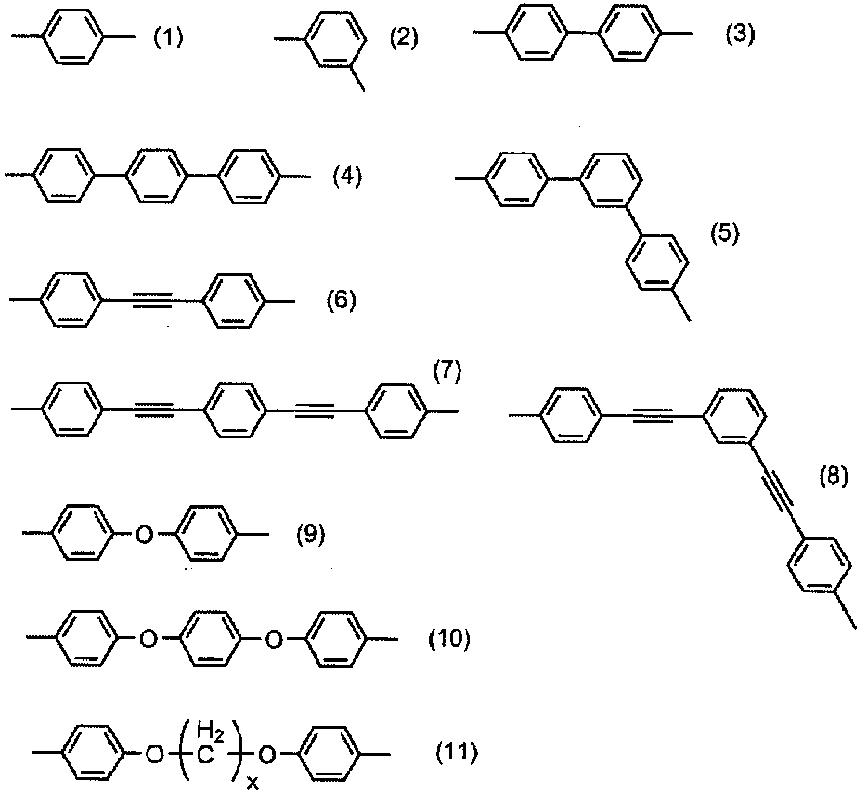

Examples of the aliphatic hydrocarbon groups constituting the spacer include C₁-C₆ alkyl groups (and more specifically, an alkyl group such as a methyl group, an ethyl group, an n-propyl group, an i-propyl group, an n-butyl group and a t-butyl group) from which one hydrogen atom has been removed. Furthermore, as the divalent organic group constituting the spacer, these groups having a substituent, including an alkyl group, such as a methyl group, an ethyl group, or a hexyl group; an alkoxy group such as a methoxy group or a butoxy group; or a halogen atom such as chlorine or bromine, may be used. The spacer is preferably an alkylene group, and more preferably a tetramethylene group (-(CH₂)₄-).

Examples of the metal ions represented by M in the general formula (I) and M¹ to M^{N} in the general formula (II) include an iron ion, a cobalt ion, a nickel ion, a zinc ion and a ruthenium ion. Of these, the metal ion is preferably a metal ion having six coordination sites, and more preferably an iron ion, a cobalt ion or a ruthenium ion. These metal ions not only can change the valence thereof due to a reduction reaction, but also individually have different oxidation-reduction potentials from each other when incorporated in the organic-metallic hybrid polymer represented by the above formula (I).

Examples of the counter anions represented by X in the general formula (I) and X¹ to X^{N} in the general formula (II) include an acetate ion, a phosphate ion, a chloride ion, a hexafluorophosphate ion, a tetrafluoroborate ion, and a polyoxometalate. Of these, an acetate ion, a phosphate ion or a tetrafluoroborate ion is preferred. The charge of metal ions is compensated by the counter anion, thereby making the organic-metallic hybrid polymer electrically neutral.
In the general formula (I), n is preferably from 2 to 10,000, and more preferably from 5 to 2,000.
In the general formula (II), n is preferably from 1 to 10,000, and more preferably from 2 to 1,000. N is preferably from 2 to 20, and more preferably from 2 to 5.

The organic-metallic hybrid polymer can be applied as a solution which is prepared by dissolving this in water or in an organic solvent. Examples of such solvents include water, methanol, ethanol, propanol, isopropanol and n-butanol. These solvents may be used alone, or two or more types thereof may be mixed for use. In particular, a mixture of methanol and isopropanol is preferred. The mixing ratio of methanol and isopropanol is preferably from 100:1 1 to 1:100, and more preferably from 10:1 1 to 1:10. As a result, the effect of improving the film formability of the polymer can be achieved.

In addition, the concentration of the organic-metallic hybrid polymer solution is preferably from 0.1 to 100 mmol/L, and more preferably from 1 to 20 mmol/L. By virtue of the above-mentioned range, the effect of improving the film formability of the polymer can be achieved.
Moreover, the thickness of the organic-metallic hybrid polymer film is preferably from 10 to 1,000 nm, and more preferably from 20 to 600 nm. By virtue of the above-mentioned range, the effect of improving the film formability of the polymer can be achieved.

For example, during the production of organic-metallic hybrid polymer represented by the general formula (I), it is possible to use a method in which a bisterpyridine derivative and a metal salt are refluxed for about 24 hours at 150°C in acetic acid or methanol. The reflux conditions may vary depending on the selected type of spacer or metal salt, although it is possible for those skilled in the art to easily select the optimum conditions.

After synthesizing the organic-metallic hybrid polymer by the method as described above, the mixture obtained by reflux may be heated to evaporate the solvent, thereby forming a powder. The powder has, for example, a purple color or the like, and is in the reduced state. Because such a powder easily dissolves in methanol, it is easy to handle.

In addition, the organic-metallic hybrid polymer represented by the general formula (II) can be produced, for example, by a method including a step of refluxing each of the bisterpyridine derivatives corresponding to the 1st to Nth represented by the general formula (II) and each of the metal salts corresponding to the 1st to Nth individually in acetic acid and methanol, and a step of mixing together the 1st to Nth (wherein N represents an integer of 2 or more) reaction products obtained in the above step.

The organic-metallic hybrid polymer itself is a known substance and is described in detail, for example, in Patent Documents 1 to 10. Hence, further descriptions therefor will be omitted.

Because the organic-metallic hybrid polymer is highly stable and reliable, is easy to process, and can easily form a uniform thin film on the glass surface, it is a much more suitable material for smart windows than other organic or inorganic substances which had been conventionally used as a light transmittance-variable material.

Specifically, the smart window of the present invention has a configuration in which the organic-metallic hybrid polymer and an electrolyte are sandwiched between two conductive transparent plates. Here, a polymer gel electrolyte is preferably used as the electrolyte.

The polymer gel electrolyte to be used herein is a gel electrolyte using an organic solvent and a polymer. As the electrolyte used in the polymer gel electrolyte, preferred is a compound which is soluble in an organic solvent and which has a satisfactory electrical conductivity (0.2 S/m or more), such as a lithium salt, a sodium salt, a potassium salt, or an ammonium salt, and examples of such compounds include lithium perchlorate, lithium tetrafluoroborate, lithium hexafluorophosphate, lithium trifluorosulfate, lithium hexafluoroarsenate, ammonium perchlorates, such as tetrabutylammonium perchlorate, tetraethylammonium perchlorate, and tetrapropylammonium perchlorate, and ammonium hexafluorophosphates, such as tetrabutylammonium hexafluorophosphate, tetraethylammonium hexafluorophosphate, and tetrapropylammonium hexafluorophosphate. Of these, a lithium salt is preferred, and lithium perchlorate, lithium tetrafluoroborate or lithium hexafluorophosphate is more preferred.

As the organic solvent, for example, an organic solvent having a boiling point within the range of 120 to 300°C can be used, so that after an electrolyte is formed, the organic solvent can remain in the electrolyte without causing volatilization. Examples of such organic solvents include propylene carbonate, ethylene carbonate, ethylmethyl carbonate, diethyl carbonate, dimethyl carbonate, butylene carbonate, γ-butyrolactone, tetramethylurea, sulfolane, dimethyl sulfoxide, 1,3-dimethyl-2-imidazolidinone, 2-(N-methyl)-2-pyrrolidinone, hexamethylphosphoric triamide, N-methylpropionamide, N,N-dimethylacetamide, N-methylacetamide, N,N-dimethylformamide, N-methylformamide, butyronitrile, propionitrile, acetonitrile, acetylacetone, 4-methyl-2-pentanone, 2-butanol, 1-butanol, 2-propanol, 1-propanol, acetic anhydride, ethyl acetate, ethyl propionate, dimethoxyethane, diethoxyfuran, tetrahydrofuran, ethylene glycol, diethylene glycol, triethylene glycol monobutyl ether, tricresyl phosphate, 2-ethylhexyl phosphate, dioctyl phthalate and dioctyl sebacate.

Of these, a cyclic carboxylate ester-based compound, such as propylene carbonate, ethylene carbonate, ethylmethyl carbonate, diethyl carbonate, dimethyl carbonate, butylene carbonate, or γ-butyrolactone, is preferably used.

As the polymer for dispersing the electrolyte, a polymer that dissolves or swells (gels) by the addition of the above-mentioned organic solvent and which has high transparency is preferred, and examples thereof include polymethacrylate esters, such as polymethyl methacrylate, polyethyl methacrylate, polybutyl methacrylate, polycyclohexyl methacrylate and polyphenyl methacrylate; and polycarbonates. Of these, polymethacrylate esters are preferred, and polymethyl methacrylate or polyethyl methacrylate is more preferred.

The smart window produced using an organic-metallic hybrid polymer is capable of retaining the current color and light transmittance for a while even after the drive signal has been turned off. In other words, it is possible to provide the aforementioned smart window with a long recovery time (i.e., a duration after the drive signal has been turned off until the smart window has regained the color and light transmittance of the steady-state), which is a property absent in the previously proposed smart windows. The recovery time varies dramatically depending on the amount of electrolyte used or the application method thereof. A more detailed description will be provided in the examples section.

It is beneficial to provide the smart window with a self-retaining property by increasing the recovery time. For example, it is possible to further reduce the power consumption of smart windows by using this property. In other words, even during use of the smart window while changing the light transmittance, instead of constantly supplying the drive power, it is possible to carry out the intermittent drive control repeating the cycle to suspend supply of the drive power for a period in which changes in the light transmittance are practically negligible, for example, for about a few seconds to tens of minutes, and then to supply the drive power for a short period of time.

In the present invention, by exploiting the low power consumption properties of the smart window which has been proposed here, it becomes possible to drive the smart window by solar cells instead of the commercial power supply. The smart window can be driven even by the solar cells with a relatively small area, since the power consumption of the smart window is low. Thus, since there is no need to occupy a large area on the outer wall of the building with solar panels, the handling of buildings in terms of exterior design also becomes easy. In addition, since the power consumption of the smart window is low, it can be used indoors and places away from direct sunlight. Furthermore, even in the furniture and fixtures where power wires may move about and become an obstacle, it is possible to drive a smart window using the power generated by the solar cells due to the indoor lighting.
Needless to say, other power sources such as secondary batteries can also be prepared for nighttime or the like during which power generation is not possible.
The present invention will be described below in further detail based on a series of examples.

### [Examples]

### [Production of smart window]

In the present example, a Fe(II)-organic-metallic hybrid polymer is synthesized and used as a light transmittance-variable material, although the polymer is not limited thereto.

The synthesis thereof was carried out as follows.

Equimolar amounts of ligands, that is, bis(terpyridine) represented by the following structural formula and iron acetate (Fe (OAc)₂), were refluxed for 24 hours in CH₃COOH (about 1 mL of solvent for 1 mg of ligand) in which oxygen had been removed from the solvent by bubbling argon for a long period of time. Then, the reaction solution was cooled to room temperature and a small amount of insoluble residues was removed by filtration. The filtered liquid was transferred to a petri dish and dried by slowly evaporating the solvent. The fragile film remained in the petri dish was collected and further dried overnight in a vacuum, thereby obtaining a Fe(II)-organic-metallic hybrid polymer (yield of 90% or more) represented by the following structural formula.

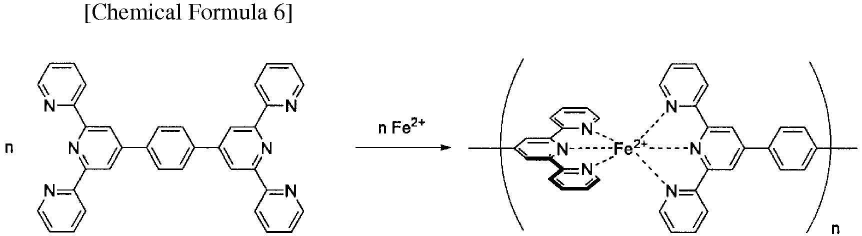

Then, a smart window was prepared by using the Fe(II)-organic-metallic hybrid polymer obtained in this manner.

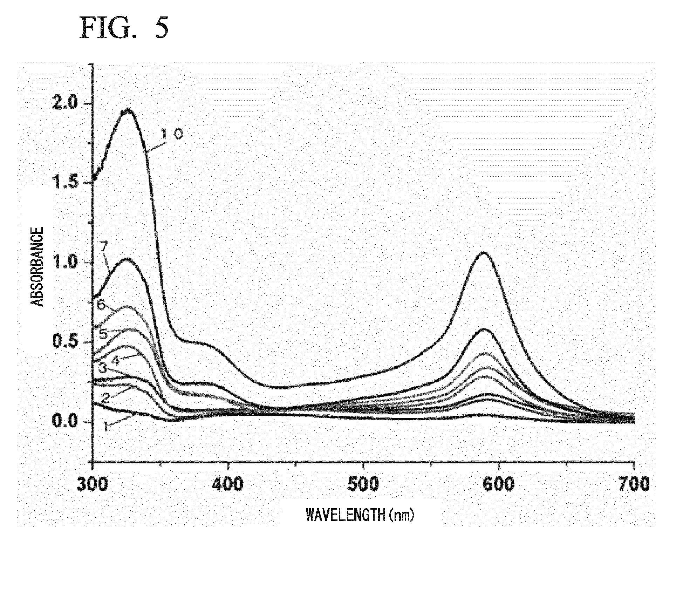

In the application of organic-metallic hybrid polymer to the transparent plates, a solution prepared by dissolving the organic-metallic hybrid polymer in a 1:1 mixture of methanol and isopropanol was spin-coated. Experiments were conducted by changing the concentration of organic-metallic hybrid polymer from 1.0 mmol/L, 2.0 mmol/L, 3.0 mmol/L, 4.0 mmol/L, 5.0 mmol/L, 6.0 mmol/L, 7.1 mmol/L to 10.0 mmol/L.
Note that in the graphs in FIG. 5 and FIGS. 7 to 10, the curves corresponding to the smart windows produced using the organic-metallic hybrid polymers having the following concentrations are represented by the following numbers.
1: 1.0 mmol/L;
2: 2.0 mmol/L;
3: 3.0 mmol/L;
4: 4.0 mmol/L;
5: 5.0 mmol/L;
6: 6.0 mmol/L;
7: 7.1 mmol/L;
10: 10.0 mmol/L.

Here, the operation program for the spin coating device was set as follows.
1. Accelerate from the stationary state to 100 rpm within 5 seconds
2. Maintain at 100 rpm for 20 seconds
3. Accelerate to 130 rpm within 5 seconds
4. Maintain at 130 rpm for 30 seconds
5. Accelerate to 150 rpm within 5 seconds
6. Maintain to 150 rpm for 90 seconds

In the present example, the ITO glass having a size of 20 cm x 20 cm was used as two sheets of transparent plates.

One ITO glass was fixed to the plate of the spin coating device, and 10 mL of the above organic-metallic hybrid polymer solution was supplied onto the surface of the ITO film in the ITO glass so that this solution covered the entire surface of the ITO film in the ITO glass. Then, spin coating was carried out in accordance with the above program. Thereafter, the resultant was dried at room temperature for 5 minutes. The ITO glass in which the ITO film side was coated with the organic-metallic hybrid polymer was produced in this manner.

In addition, a gel electrolyte was prepared by mixing the following components.
PMMA (polymethyl methacrylate) 2.1 g
Propylene carbonate 6 mL
Lithium perchlorate 0.9 g
Acetonitrile 27 mL

An organic-metallic hybrid polymer film was formed by applying the organic-metallic hybrid polymer as described above. The gel electrolyte was applied on the aforementioned organic-metallic hybrid polymer film formed on top of the ITO glass. Furthermore, the gel electrolyte was applied to the ITO film side of the other untreated ITO glass in the same manner.

It should be noted that although the gel electrolyte was prepared as described above, this does not mean that the total amount thereof is applied to two sheets of glass having a size of 20 cm x 20 cm. With regard to the electrolyte gel to be prepared, the ratio between the components is more important than the absolute amount.

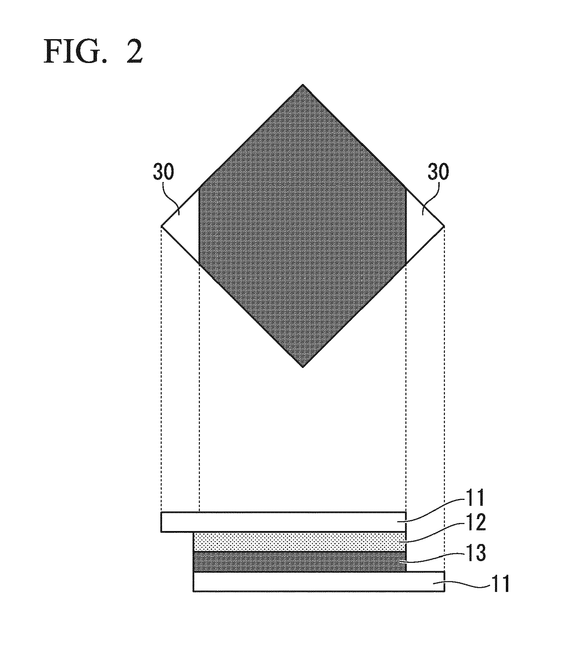

Two sheets of glass coated with the gel electrolyte were allowed to stand for 10 minutes, and then the surfaces covered with the gel electrolyte were made to face each other to adhere, thereby obtaining a solid-state device shown in FIG. 2. In FIG. 2, one corner of these ITO glasses is cut away into a triangular shape. It should be noted that this is for preparing a condition so that, by combining these glasses as shown in FIG. 2, one corner of each glass is not covered by the other glass as shown below in the same drawing, and then attaching electrodes to each of these two triangular shaped portions. Drive voltage is applied by these electrodes to the organic-metallic hybrid polymer and gel electrolyte sandwiched between two sheets of glass.

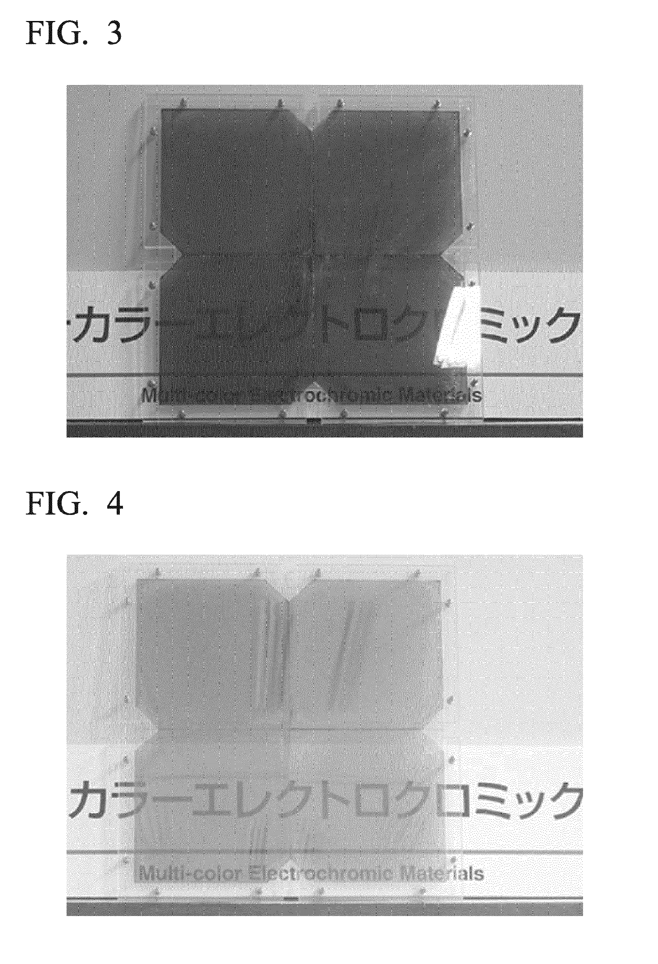

Regarding the smart window produced in this manner, FIG. 3 is a photograph showing the state thereof at the time of coloration and FIG. 4 is a photograph showing the state thereof at the time of decoloration. In the photographs in FIGS. 3 and 4, a large smart window having a size of 40 cm x 40 cm is constructed by vertically and horizontally aligning and combining 4 sheets of the 20 cm x 20 cm smart window prepared as described above. In addition, in order to visually show the concentrations of the colored state and colorless state more clearly, a piece of paper with written characters or the like has been placed behind the smart window. In FIG. 3, the smart window is colored in purple. From these figures, it is clear that the color (transmittance) varies considerably by the application of voltage in the smart window of the present invention.

### [Measurements of characteristics of smart window]

When measuring the characteristics of solid-state device obtained in this manner, for the convenience of the measurements, a small device having a size of 38 mm x 10 mm was produced by the method described above. Solid-state devices for measurements were produced by the same conditions with the exception that the aforementioned organic-metallic hybrid polymer solutions with various concentrations were used in order to change the thickness of the organic-metallic hybrid polymer film. Further, in the measurements, a voltage of 3V was applied between two sheets of ITO glass using a battery as a power source. The measurement results are as follows.

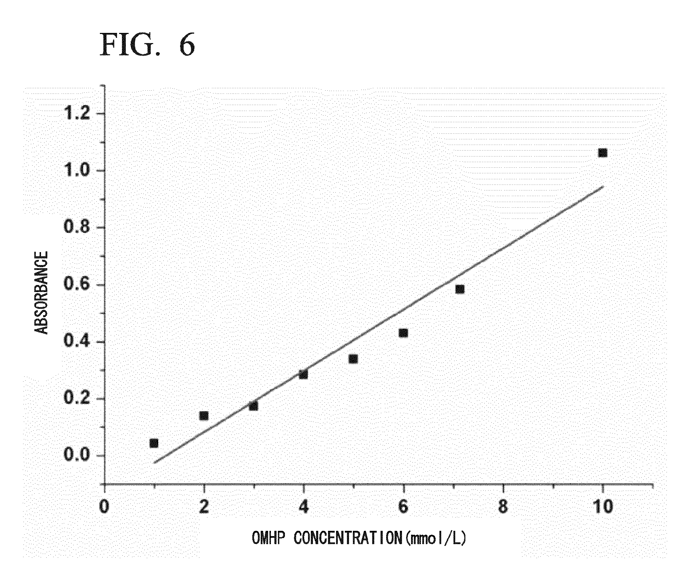

FIG. 5 shows the ultraviolet-visible absorption spectrum of these solid-state devices. An almost linear relationship is established between the ultraviolet-visible absorption at 581 nm (specific absorbance by the Fe-organic-metallic hybrid polymer) and the concentration of the organic-metallic hybrid polymer solution that was used to prepare solid-state devices. For clarification, the relationship between them is shown in FIG. 6.

According to the Lambert-Beer law, the ultraviolet-visible absorption spectrum in this case reflect the thickness of the film. Therefore, under the same production program, the relative thickness of the organic-metallic hybrid polymer film has a linear relationship with the concentration of the organic-metallic hybrid polymer solution.

It was found that the thickness of the films produced with the organic-metallic hybrid polymer solutions having a concentration of 1.0 to 10.0 mmol/L was substantially 20 nm to 300 nm when measured using a surface profiler.

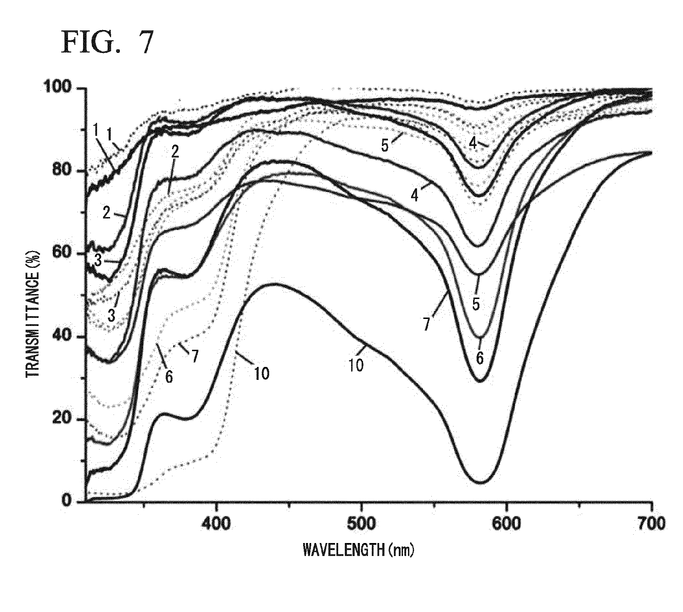

When the voltage applied to these solid-state devices was changed, a change of color from blue due to reduction and a change of color from colorless due to oxidation were clearly observed. FIG. 7 shows the change in the ultraviolet-visible absorption spectrum due to the voltage. In FIG. 7, the curve drawn with the solid line indicates the spectrum when the applied voltage is 0 volts and the dashed line indicates the spectrum when the applied voltage is 3 volts.

Compared to the case where the applied voltage is 3 volts, the light transmittance at 581 nm, in particular, swings in the opposite direction when the applied voltage is 0 volts. In other words, the applied voltage of 0 volts results in a low transmittance and a colored state, whereas the applied voltage of 3 volts results in a high transmittance and a colorless state. The smart window of the present invention takes advantage of these color change characteristics.

Then, the color change response time of the produced solid-state devices was measured. For this measurement, the drive voltage supplied from the battery was changed in two stages from -3 volts to 3 volts while the measurement was conducted by irradiating the light having a wavelength of 581 nm onto the solid-state device.

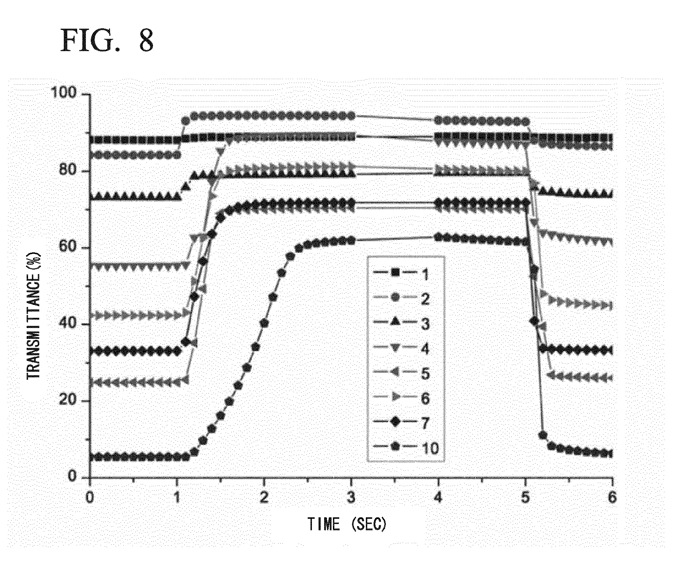

The graph illustrated in FIG. 8 shows the measurement results for the change in the light transmittance over time when the applied voltage was set to -3 volts during the period from 0 seconds to 1 second, changed to +3 volts during the period from 1 second to 5 seconds, and finally returned to -3 volts during the period from 5 seconds to 6 seconds.

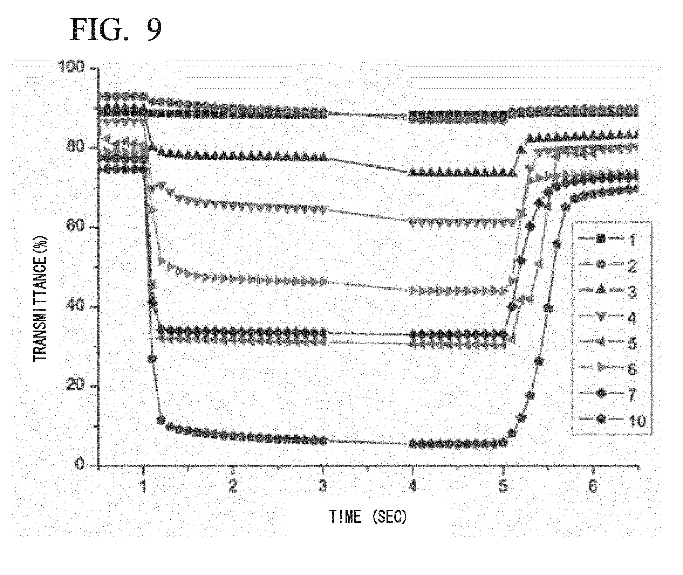

The graph illustrated in FIG. 9 shows the measurement results when the applied voltage was changed to +3 volts, -3 volts, and then to +3 volts at the same timing as that of FIG. 8.

From these measurement results, when the organic-metallic hybrid polymer is relatively thick (in other words, when the concentration of organic-metallic hybrid polymer is relatively high), it is apparent that the rate of change from blue to colorless is significantly slower than the rate of change from colorless to blue.

Then, the memory effect (self-retaining property) of solid-state devices was examined.

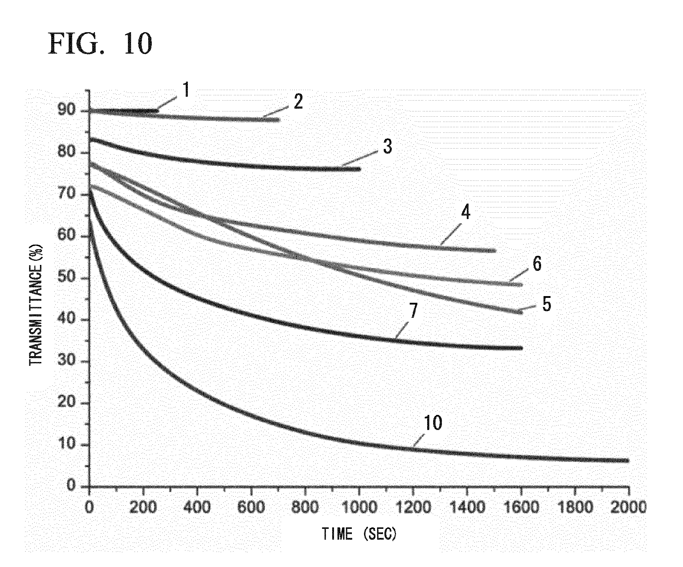

FIG. 10 is a graph showing the results of temporal change in the light transmittance of the solid-state devices using a light having a wavelength of 581 nm which were measured by turning the solid-state devices colorless by applying a drive voltage thereto, and then adjusting the applied voltage to 0 volts at a time point of 0 seconds. As can be seen from this graph, after turning off the drive voltage, it takes 20 to 30 minutes or more in order to stabilize the light transmittance of the solid-state device.

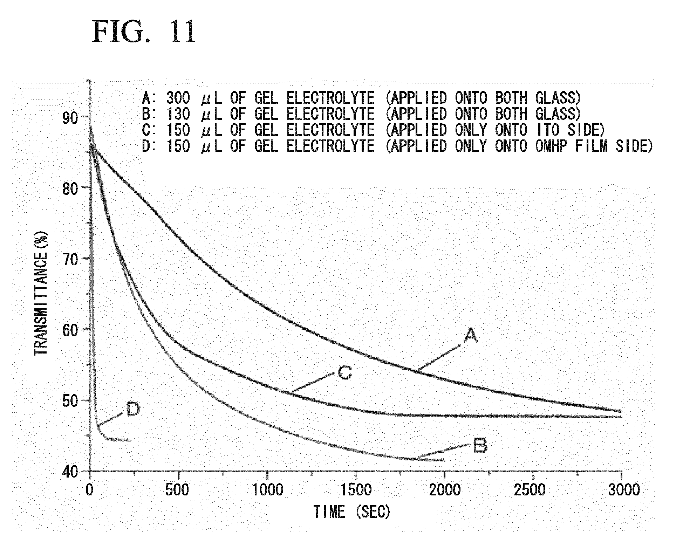

The decrease rate of light transmittance varies considerably in accordance with the amount of electrolyte and the application method thereof. FIG. 11 is a graph showing the results measured by the same method as in FIG. 10, indicating that the rate of decrease in transmittance changes when these conditions to be measured were altered. As the measuring object, a 20 cm x 20 cm device was produced using the same method as the method for producing solid-state devices as described above.
The curve in FIG. 11 indicates the decrease rate of light transmittance in the following solid-state devices.
Curve A: a solid-state device produced by applying 300 µL of gel electrolyte to both the ITO glass formed with an organic-metallic hybrid polymer film and another piece of unprocessed ITO glass;
Curve A: a solid-state device produced by applying 130 µL of gel electrolyte to both the ITO glass formed with an organic-metallic hybrid polymer film and another piece of unprocessed ITO glass;
Curve C: a solid-state device produced by applying 150 µL of gel electrolyte only onto the ITO film of unprocessed ITO glass; and
Curve D: a solid-state device produced by applying 150 µL of gel electrolyte only onto the organic-metallic hybrid polymer film formed on top of the ITO glass.

As can be seen clearly from FIG. 11, when the gel electrolyte is applied to both of the ITO glass, although the ultimate transmittance is almost the same, the rate of change is slower when a large amount of gel electrolyte is used, resulting in a larger memory effect.

In particular, in the case of curve A, because the decrease in transmittance immediately after stopping the application of the drive voltage is slow, it is convenient when used in the window or the like. In other words, after stopping the application of drive voltage, it has taken 5 minutes or more for the transmittance to decline by 10% in the curve A. In general, even when the overall brightness gradually declines by about 10%, people do not notice unless they are closely monitoring. Accordingly, during this period, there is no need to supply a drive voltage to the aforementioned smart window. Therefore, when a decrease in the transmittance immediately after the application of the drive voltage is small, as seen in the curve A, intermittent drive with longer cycle time can be carried out, and thus the power required for driving can be saved considerably.
It should be noted that when the drive voltage in the next cycle is supplied from the drive circuit after a predetermined idle period has elapsed, if driven at the rated voltage from the beginning, the transmittance of the smart window drops to the initial value within a short period of time (i.e., about 0.1 seconds). Therefore, it may be possible to visually recognize the changes in the transmittance depending on the usage or the setting of the driving cycle period. In such a case, the drive voltage may be controlled so as to increase up to the rated value with a time constant of a few seconds to few tens of seconds. If the time constant is too short, people can visually recognize the changes in the transmittance, whereas if the time constant is too long, the driving power increases.

In addition, when the gel electrolyte was applied only to one ITO glass (curves C and D), the rate of change is significantly faster than the case where the gel electrolyte was applied to both of the ITO glass. The memory effect is hardly observed especially when the gel electrolyte is applied only to the organic-metallic hybrid polymer film side (curve D), that is, when the adhesion between the gel electrolyte and the ITO film is not sufficient.

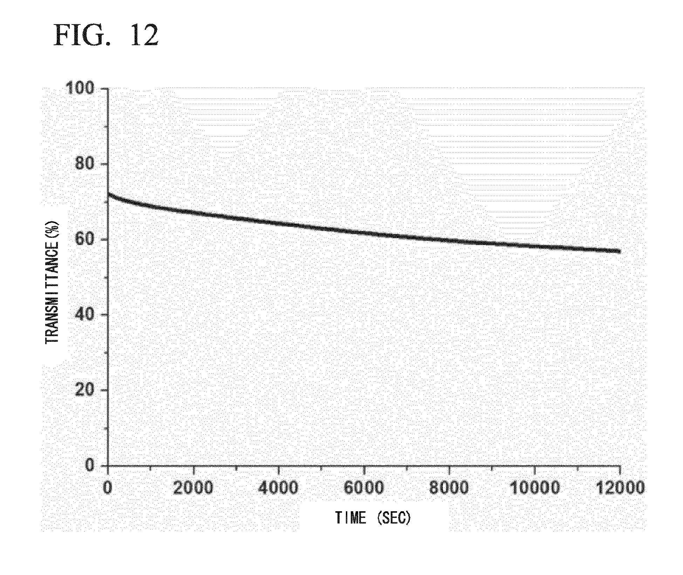

When the thickness of the gel electrolyte is further increased, the memory effect further increases. FIG. 12 is a diagram showing a temporal variation in the transmittance of the solid-state devices. Here, the aforementioned solid-state devices were produced in the same manner as the smart window corresponding to the curve A in FIG. 11, with the exception that the gel electrolyte was applied to both of the ITO glass so that the thickness of the gel electrolyte layer sandwiched between two pieces of ITO glass was 6 mm. Within the time range shown in FIG. 12, the downward trend of transmittance has continued even at the end of the graph on the right, and the transmittance was ultimately reduced to 38%. When the transmittance decreases from the maximum transmittance at time 0 seconds and stabilizes at the minimum transmittance, if the time required for reaching 50% of this range of variation is defined as the time constant for the memory effect, the time constant of the solid-state devices of which a transmittance change is shown in FIG. 12 is more than 3 hours (100,800 seconds). The reason why the memory effect increases when the thickness of the gel electrolyte layer is increased as described above is considered as follows. The distance electrons need to migrate within the gel electrolyte, in order to make the metal ions involved in the coloration (in this case, Fe(III) ions) return to a reduced state, is increased, and thus reduction becomes more difficult. As described above, in order to achieve a memory effect for a very long period of time, it is preferable to set the thickness of the gel electrolyte layer within a range of 1 mm to 10 mm.

### [Smart window system]

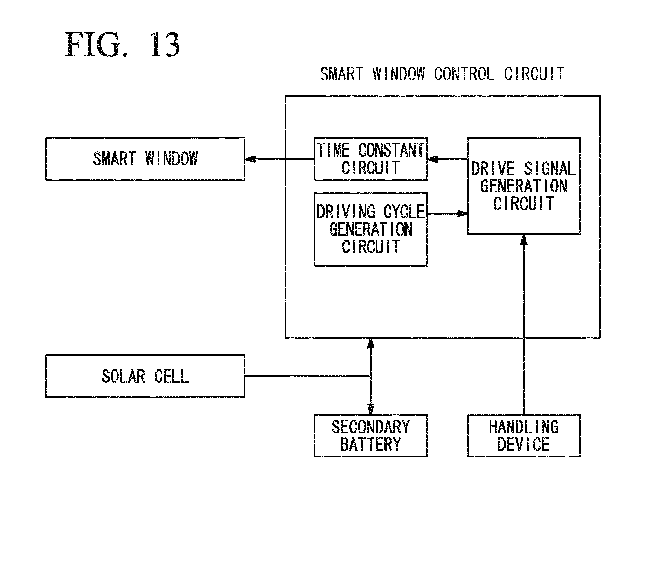

FIG. 13 shows a conceptual diagram of a smart window system in which the smart window of the present invention is applied to a window of a building or the like.

In FIG. 13, the smart window is installed in a window frame (not shown) of a building or the like. In addition, in order to supply the driving power for the smart window, solar cells are installed on the outer wall of the building or the like (not shown). Further, the power obtained from solar cells is used to charge a secondary battery, and when a smart window control circuit is operating, the power is supplied from the secondary battery, or from both the secondary battery and solar cells.

The location for installing the solar cells is not particularly limited, as long as it is convenient for installation and is also a place where enough daylight can be collected for supplying the required power. Further, instead of being provided as a separate body, it is also possible to configure the solar cells integrally with the smart window, for example, by embedding them in a portion of the window frame.

The smart window system is also provided with a handling device. The user can modify various settings, for example, to increase or decrease the light transmittance of the smart window using the handling device.

A drive signal generation circuit within the smart window control circuit provides a time constant circuit with a control signal indicating the voltage to be applied to the smart window based on a periodic signal from a driving cycle generation circuit. This driving cycle generation circuit is a circuit for generating a periodic timing signal in order to generate an intermittent drive signal by using the memory effect of the smart window which has been already mentioned. As a result, it is possible to maintain the light transmittance of the smart window at a substantially constant level as seen through the human eye, and also to reduce the power consumption.

The time constant circuit functions as a driver for the smart window. However, in a state where the light transmittance of the smart window is maintained at a substantially constant level as seen through the human eye, the aforementioned problem arises when the drive voltage of the smart window is increased immediately from the idle state to the rated value. In order to solve this problem, it may also be controlled for slowly increasing the drive voltage with a time constant of a few seconds to few tens of seconds.

Because of such configurations, the smart window system shown in FIG. 13 does not require power from a commercial power supply, and the routing of the power line from a commercial power supply during installation of the smart window is not required either.

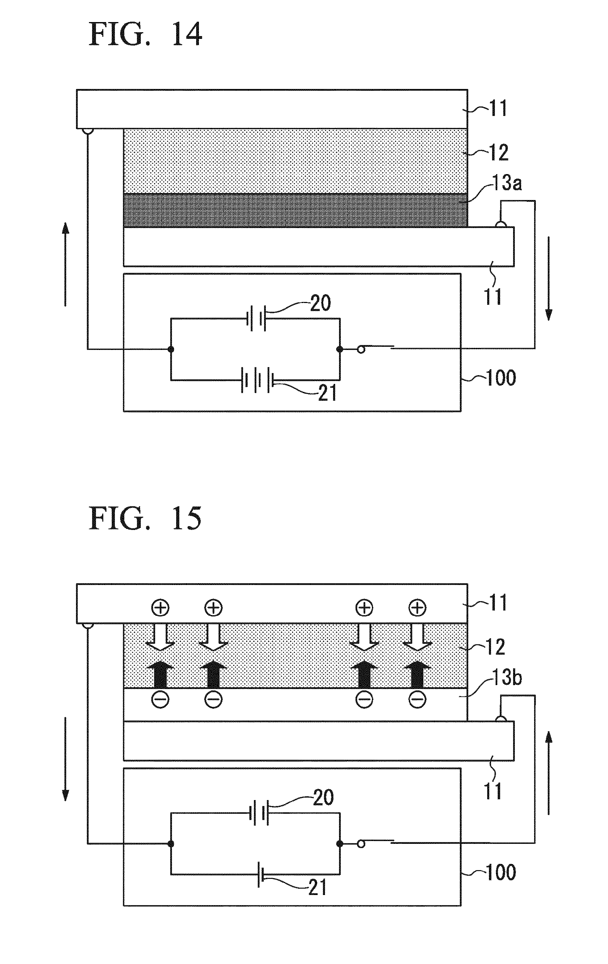

FIGS. 14 and 15 show conceptual diagrams of the smart window system of the present invention in other Examples using a solar cell. In this smart window system, through an extremely simple control, it is possible to decrease the transmittance of the smart window when subjected to direct sunlight, and conversely, to quickly increase the transmittance if it is no longer exposed to direct sunlight when the sun is hidden behind the clouds. As a result, it is possible to reduce the impact of changes in the sunshine outdoors on the brightness of the room as much as possible.

In FIGS. 14 and 15, a drive signal is supplied from an external light-sensitive drive unit to the smart window having the above-mentioned structure in which the organic-metallic hybrid polymer film and gel electrolyte are sandwiched between two sheets of ITO glass. In the external light-sensitive drive unit, as shown in the drawing, the solar cell and an opposing battery which is connected in anti-parallel therewith (i.e., connected in parallel while the polarity is in the reverse direction) are provided.

An output voltage of 3.4 V can be achieved when strong light such as direct sunlight is irradiated onto the solar cell, whereas an output voltage of only about 0.8 V (or even lower voltage depending on the brightness) is achieved when the solar cell is placed in a shaded area. On the other hand, the opposing battery supplies a voltage of 3 V in the opposite direction to the electromotive force of the solar cell. By configuring the external light-sensitive drive unit in such a manner, the smart window system in FIGS. 14 and 15 is capable of achieving the effects of changing the transmittance of light in response to the strength of the sunshine in real time and reducing changes in the amount of light coming into the building through this smart window with a simple configuration even if the amount of sunlight irradiated onto the smart window changes significantly depending on the sunshine conditions.

In other words, at the time of direct sunlight irradiation as shown in FIG. 14, in order to generate a voltage of 3.4 V which is higher than the voltage of 3V generated by the opposing battery, the solar cell counteracts the reverse voltage of the opposing battery and also applies a voltage of 0.4V to the smart window in the direction indicated by the arrow. As a result, the smart window develops a color as described above to reduce the intensity of direct sunlight entering inside the building.

On the other hand, as shown in FIG. 15, when the sun is blocked by the clouds or the like, because the voltage generated by the solar cell drops to about 0.8V, the voltage of the opposing battery can no longer be cancelled out completely, and thus a voltage of about 2.2V is applied to the smart window in the opposite direction to that at the time of direct sunlight irradiation as indicated by the arrow. As a result, the smart window becomes colorless because the metal ions within the organic-metallic hybrid polymer (OMHP) film are oxidized, and by reducing the attenuation of external light upon entering inside the building, the extent of illuminance reduction inside the building is reduced. Thus, changes in the illuminance inside the building due to changes in the sunlight or the like can be reduced.

It should be noted that the changes in the illuminance, when the sun is hidden behind the clouds and then reappears, may occur very rapidly. In order to follow such rapid changes in the illuminance, it is desirable that responsiveness of the transmittance of the smart window used in the present Example to the drive signal be fast.

It should be noted that the opposing battery may be a battery of any kind as long as it is fully capable of driving a smart window. In addition, although it is represented as a battery in the conceptual diagram, since this may be any power source to substantially act as a direct current constant voltage source, it may be a power supply circuit or the like to operate by the power supplied from the external power source. Further, although not shown in the drawings, a charging circuit in order to avoid the draining of opposing battery, a switching circuit for switching to another battery or the like may also be provided. Furthermore, it is desirable to adjust the level of the sunlight irradiation received by the solar cell to the same level as that of the irradiation received by the smart window as much as possible by installing the solar cell in the vicinity of the smart window or at the edge portion of the smart window or the like. In addition, although the solar cell has been collectively installed together in one place in the drawings, it can also be configured so that a plurality of solar cells are distributed in multiple locations around and at the edge of the smart window, and any one of solar cells is irradiated with direct sunlight even when, for example, the direct sunlight is irradiated only in the upper half of the window, thereby improving the accuracy of the transmittance control of the smart window.

### [Industrial Applicability]

As described above in detail, with the smart window of the present invention, a product with a large area can be produced more easily, as compared to the smart window which has been previously proposed, and together with the memory effect thereof, large energy savings can also be achieved by using a solar cell in combination. Therefore, a great deal of industrial applicability can be expected.

### [Reference Signs List]

11: ITO glass;
12: Gel electrolyte layer;
13: OMHP film;
13a: OMHP film at the time of coloration;
13b: OMHP film at the time of decoloration;
20: Opposing battery;
21: Solar cell;
30: Electrode mounting unit;
100: External light-sensitive drive unit

## Claims

1. A smart window in which an organic-metallic hybrid polymer and an electrolyte are sandwiched between two conductive transparent plates.

2. The smart window according to Claim 1, wherein said organic-metallic hybrid polymer is a polymer represented by the following general formula (I) or (II): 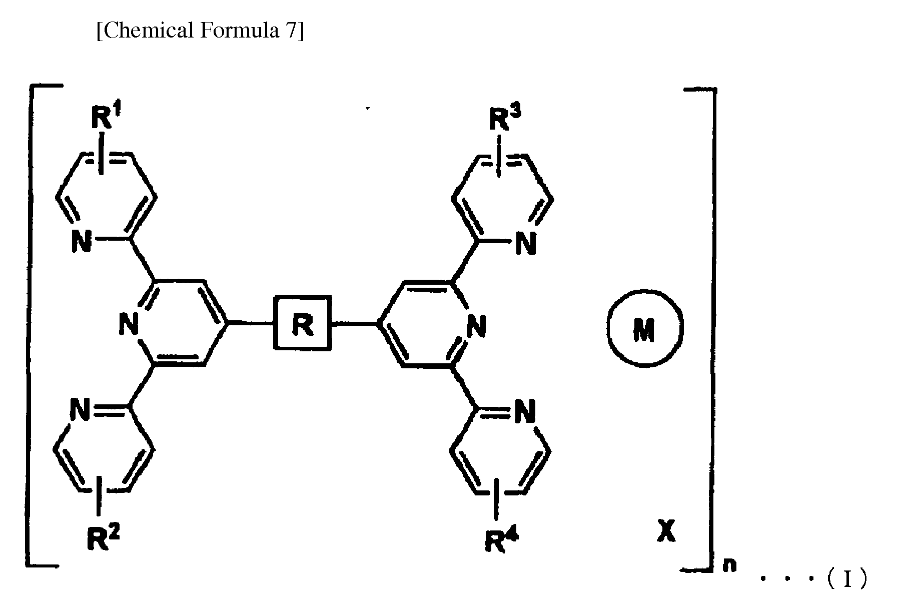 wherein M represents a metal ion; X represents a counter anion; R represents a spacer containing a carbon atom and a hydrogen atom, or a spacer directly connecting two terpyridyl groups; each of R¹ to R⁴ independently represents a hydrogen atom or a substituent; and n represents an integer of 2 or more which indicates a degree of polymerization; 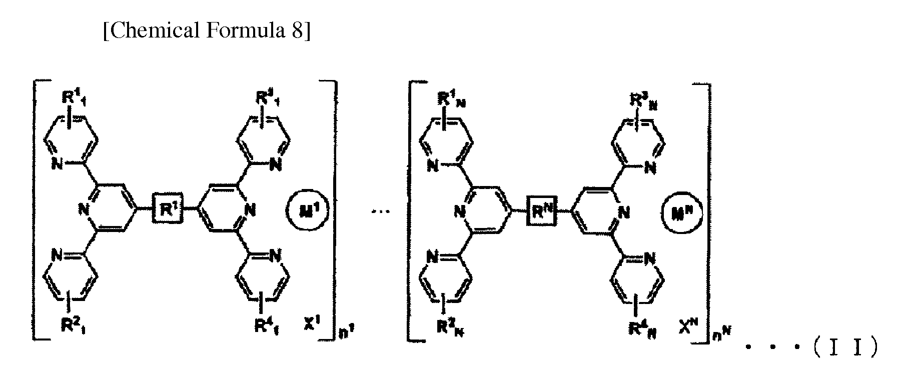 wherein each of M¹ to M^{N} (wherein N represents an integer of 2 or more) independently represents a metal ion; each of X¹ to X^{N} (wherein N represents an integer of 2 or more) independently represents a counter anion, each of R¹ to R^{N} (wherein N represents an integer of 2 or more) independently represents a spacer containing a carbon atom and a hydrogen atom, or a spacer directly connecting two terpyridyl groups; each of R¹₁ to R¹_{N}, R²₁ to R²_{N}, R³₁ to R³_{N}, and R⁴₁ to R⁴_{N} (wherein N represents an integer of 2 or more) independently represents a hydrogen atom or a substituent; and each of n¹ to n^{N} (wherein N represents an integer of 2 or more) independently represents an integer of 2 or more which indicates a degree of polymerization.

3. The smart window according to Claim 1 or 2, wherein said conductive transparent plate is a glass plate having a conductive thin film formed on the surface thereof.

4. The smart window according to any one of Claims 1 to 3, wherein said transparent plate is an ITO glass.

5. The smart window according to any one of Claims 1 to 4,
wherein said organic-metallic hybrid polymer sandwiched between said two transparent plates is applied on top of said transparent plate by spin coating a solution of organic-metallic hybrid polymer on one of said two transparent plates.

6. The smart window according to Claim 5,
wherein said solution of organic-metallic hybrid polymer is a solution prepared by dissolving the organic-metallic hybrid polymer in a mixture of methanol and isopropanol.

7. The smart window according to any one of Claims 1 to 6, wherein said electrolyte is a conductive gel.

8. The smart window according to any one of Claims 1 to 7, wherein a thickness of said electrolyte between said two transparent plates is from 1 mm to 10 mm.

9. The smart window according to any one of Claims 1 to 8, wherein said electrolyte contains lithium perchlorate.

10. A method of producing the smart window of any one of Claims 5 to 8, the method comprising:
applying said electrolyte to both said organic-metallic hybrid polymer on top of said transparent plate and an ITO film of one of said transparent plates to which said organic-metallic hybrid polymer has not been applied; and
aligning and adhering surfaces of said two transparent plates to which said electrolyte has been applied, thereby producing the smart window.

11. A smart window system comprising:
the smart window of any one of Claims 1 to 9; and
a drive circuit for intermittently applying a drive voltage to said two transparent electrode plates.

12. The smart window system according to Claim 11, further comprising a solar cell which supplies power to said drive circuit.

13. A smart window system comprising:
the smart window of any one of Claims 1 to 9;
a solar cell and a direct current power supply which are connected in parallel and in opposite directions from each other; and
an external light-sensitive drive unit which supplies a drive signal to said smart window,
wherein transmittance of said smart window is changed in a direction so as to counteract an illuminance change of light outside.
